# EUROPÄISCHE PATENTSCHRIFT

(11) **EP 1 081 493 B1**
(45) Veröffentlichungstag und Bekanntmachung des Hinweises auf die Patenterteilung: **02.11.2006**
(21) Anmeldenummer: 99116991.3
(22) Anmeldetag: 28.08.1999
(51) Int. Cl.: G01N 33/00

(54) **Gasmelder mit Druckkapselung für Explosionsschutz**
Gas detection device with an explosion-proof casing
Détecteur de gaz à enveloppe antidéflagrante

(43) Veröffentlichungstag der Anmeldung: 07.03.2001
(73) Patentinhaber: Siemens Schweiz AG, 8047 Zürich (CH)
(72) Erfinder: Pleisch, Rolf, 8604 Volketswil (CH)
(74) Vertreter: Weise, Wolfgang

(56) Entgegenhaltungen:
- EP-A- 0 016 351
- DE-A- 2 035 173
- DE-A- 3 810 409
- US-A- 3 476 517

## Beschreibung

Die vorliegende Erfindung betrifft einen Gasmelder mit Druckkapselung für Explosionsschutz, mit einem einen Sensor enthaltenden Sensorgehäuse und mit einer als Flammensperre ausgebildeten Gehäuseabdeckung zur Abschirmung des Sensors gegen die Umgebung und zur Ermöglichung eines Gasaustausches mit dieser.

Als Sensoren werden heute weitgehend entweder elektrisch beheizte Thermoelemente (Pellistoren) oder Metalloxid-Halbleiter oder elektrochemische Sensoren verwendet, wobei bei den Thermoelementen und den Metalloxid-Halbleitern die Arbeitstemperatur in einem Temperaturbereich liegt, in dem die Entzündungstemperatur der nachzuweisenden Gase (Aether und Acetaldehyd 180°C, Benzin und Dieselkraftstoff 220-300°, Acetylen 305°, usw.) bereits weit überschritten ist. Aus diesem Grund müssen Massnahmen getroffen werden, welche verhindern, dass die Gasmelder die Entzündung der Gase bewirken, vor deren Anwesenheit sie warnen sollen. Diese Schutzart wird als Ex-d bezeichnet. Ausserdem müssen derartige Gasmelder darüber hinaus noch bestimmte Anforderungen bezüglich Prüfdruck erfüllen.

Die heute verwendeten Flammensperren (siehe beispielsweise EP-A-0 032 844) sind Filterscheiben aus einem metallischen Sinterwerkstoff wie Cr-Ni-Stahl mit einer Porengrösse von etwa 30-40 µm, welche aber für gewisse toxische, bereits in geringen Spuren nachzuweisende Gase, wie beispielsweise Chlor und Ammoniak wegen der starken Adsorptionswirkung nicht angewendet werden können. Wegen der kleinen Signale und der langen Austauschzeiten müssen die Sinterwerkstoffe heute weggelassen werden, was aber einen Einsatz der betreffenden Gasmelder in explosionsgefährdeten Zonen verunmöglicht.

Andererseits ist es aber auch nicht möglich, als Flammensperre nur Metallgitter ohne Sintereinlage zu verwenden (siehe beispielsweise EP-A-0 764 847), weil diese keine ausreichende Abschirmung des Sensors gegen Verschmutzung gewährleisten.

Durch die Erfindung soll nun eine Flammensperre angegeben werden, die einen Einsatz der Gasmelder in explosionsgefährdeten Zonen bei gleichzeitig ausreichender Abschirmung des Sensors ermöglicht.

Diese Aufgabe wird erfindungsgemäss durch einen Gasmelder nach Anspruch 1 gelöst, wobei die Gehäuseabdeckung durch ein Filter aus Metallfaservlies und ein Stützgitter gebildet ist.

Praktische Untersuchungen haben gezeigt, dass beim erfindungsgemässen Gasmelder sowohl der Signalhub (Austauschzeit) als auch die Festigkeit der Flammensperre ausserordentlich gut sind, so dass einerseits der Nachweis von Spurengasen in kurzer Zeit und andererseits der Einsatz der Melder in explosionsgefährdeten Zonen möglich ist.

Eine erste bevorzugte Ausführungsform des erfindungsgemässen Gasmelders ist dadurch gekennzeichnet, dass das Metallfaservlies aus Chrom-Nickel-Stahl besteht. Eine zweite bevorzugte Ausführungsform ist dadurch gekennzeichnet, dass das Stützgitter aus Chrom-Nickel-Stahl-Draht besteht. Gemäss einer dritten bevorzugten Ausführungsform des erfindungsgemässen Gasmelders ist das Stützgitter an der Aussenseite der Gehäuseabdeckung angeordnet und zusammen mit dem Metallfaservlies von einem Ring eingefasst.

Eine weitere bevorzugte Ausführungsform des erfindungsgemässen Gasmelders ist dadurch gekennzeichnet, dass in das Metallfaservlies ein zusätzliches Stützgitter integriert ist. Vorzugsweise sind das Schutzgitter und das Metallfaservlies in den genannten Ring eingegossen, eingeklebt oder eingespritzt. Der Ring mit der Gehäuseabdeckung ist in das Gehäuse eingepresst oder eingeklebt. Vorzugsweise besteht der Ring und der diesen tragende Gehäuseteil aus PPO NORYL.

Das Metallfaservlies weist eine Dicke von 1 mm und eine Porosität von 50 µm auf und das Stützgitter ist durch ein Quadratmaschengewebe mit einer Dicke von knapp 1 mm und einer Maschenweite von 0.5 bis 0.7 mm gebildet.

Im folgenden wird die Erfindung anhand eines Ausführungsbeispiels und der Zeichnungen näher erläutert; es zeigt:
Fig. 1 einen Schnitt durch das Sensorgehäuse eines Gasmelders; und
Fig. 2 ein Detail von Fig. 1.

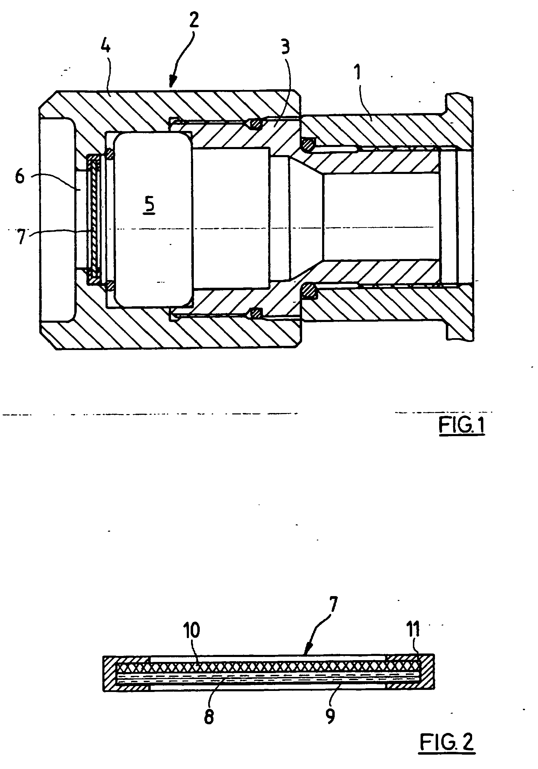

Fig. 1 zeigt einen schematischen Schnitt durch das Sensorgehäuse eines Gasmelders, welcher ein nicht dargestelltes, druckfestes Meldergehäuse aus Aluminium aufweist, in welchem die Melderelektronik eingebaut ist und welches einen Stutzen 1 aufweist, in welchen das Sensorgehäuse 2 eingeschraubt ist. Das Sensorgehäuse 2, weist ein in den Stutzen 1 geschraubtes Unterteil 3 und ein mit diesem verschraubtes Oberteil 4 auf, welch letzteres auch als Schutzkappe bezeichnet wird. Zwischen dem Unterteil 3 und dem Oberteil 4 ist ein elektrochemischer Gassensor 5 zum Nachweis toxischer Gase wie beispielsweise CO, CO₂, NO, HCl, NH₃, NO₂, oder Cl₂ angeordnet. Der Gasmelder als solcher und auch der Sensor 5 bilden nicht Gegenstand der vorliegenden Anmeldung und sind daher hier nicht näher beschrieben. Es wird in diesem Zusammenhang auf die Gasmelder der Reihe CerGas DE60 und DE64 der Siemens Building Technolgies AG, Cerberus Division (früher Cerberus AG) verwiesen (CerGas ist ein registriertes Warenzeichen der Siemens Building Technologies AG).

Der Gehäuseoberteil 4 weist an seiner vom Gasmelder wegragenden Stirnfläche eine Öffnung 6 zur Ermöglichung des Zutritts von Gas aus dem Aussenraum zum Gassensor 5 auf. Die Öffnung 6 ist durch eine Flammensperre 7 zur Verhinderung des Überschlagens von Flammen aus dem Innenraum des Sensorgehäuses 2 nach aussen abgedeckt.

Fig. 2 zeigt einen Schnitt durch die Flammensperre 7, die ausserdem eine den Sensor 5 vor Verschmutzungen schützende Abdeckung bildet und eine Diffusionsbarriere darstellt, die aber selbstverständlich die Fähigkeit des Gasmelders zur Detektion geringer Konzentrationen von toxischen Gasen nicht beeinträchtigen darf. Gemäss Fig. 2 ist die Flammensperre 7 aus zwei Schichten aufgebaut, wobei die dem Sensor 5 zugewandte Schicht durch ein Metallfaservlies 8 mit einem integrierten feineren Stützgitter 9 nach der Sensorseite und die dem Aussenraum zugewandte Schicht durch ein grobes Stützgitter 10 gebildet ist. Das Metallfaservlies 8 und das grobe Schutzgitter 10 sind beide durch runde Plättchen mit einem Durchmesser von etwa 25 mm gebildet, die aussen von einem Ring 11 eingefasst sind. Sowohl das Sensorgehäuse 1 als auch der Ring 11 bestehen aus PPO Noryl.

Das Metallfaservlies 8 und beide Schutzgitter 9 und 10 bestehen je aus Chrom-Nickel-Stahl 1.4404; das Metallfaservlies 8 hat eine Porengrösse von 50 µm und eine Dicke von 1.0 mm. Beim Metallfaservlies 8 werden in Abwandlung zur Pulvermetallurgie für Filterzwecke Fasern hergestellt, die mit Stärken von 4 µm bis 100 µm und einer mittleren Länge von 25 mm in gleichmässiger Dicke zu einem sogenannten Wirrfaservlies geschichtet und dann gesintert und gewälzt werden. Aufgrund der sehr hohen Porosität von bis zu 80%, also der im Vergleich zum Materialanteil sehr grossen Summe der Porenquerschnitte, hat das so hergestellte Metallfaservlies eine ausserordentlich hohe Durchströmbarkeit. Im Vergleich zum Teilchengrössenspektrum der verwendeten Metallpulver ist der Durchmesserbereich der Fasern sehr einheitlich, so dass auch die Porengrössenverteilung sehr eng ist. Diese Tatsache gewährleistet ein optimales Verhältnis von Filterfeinheit zu Durchströmbarkeit.

Das grobe Schutzgitter 10 ist ein Quadratmaschengewebe mit einer Dicke von 0.8 mm; der Drahtdurchmesser beträgt 0.4 mm und die Maschenweite 0.63 mm. Stützgitter 10 und Metallfaservlies 8 sind in den Ring 11 eingegossen, eingeklebt oder eingespritzt, und der Ring 11 mit dem Metallfaservlies 8 und dem Stützgitter 10, also die Flammensperre 7, ist in das Gehäuseoberteil 4 eingepresst oder eingeklebt.

Die angegebenen Masse und Materialien sind nicht einschränkend sondern als Beispiele für bevorzugte Ausführungsformen zu verstehen.

## Patentansprüche

1. Gasmelder mit Druckkapselung für Explosionsschutz, mit einem einen Sensor (5) enthaltenden Sensorgehäuse (2) und mit einer als Flammensperre ausgebildeten Gehäuseabdeckung (7) zur Abschirmung des Sensors (5) gegen die Umgebung und zur Ermöglichung eines Gasaustausches mit dieser, wobei die Gehäuseabdeckung (7) durch ein Filter (8) aus Metallfaservlies und ein Stützgitter (10) gebildet ist.

2. Gasmelder nach Anspruch 1, **dadurch gekennzeichnet, dass** das Metallfaservlies (8) aus Chrom-Nickel-Stahl besteht.

3. Gasmelder nach Anspruch 1 oder 2, **dadurch gekennzeichnet, dass** das Stützgitter (10) aus Chrom-Nickel-Stahl besteht.

4. Gasmelder nach Anspruch 3, **dadurch gekennzeichnet, dass** das Stützgitter (10) an der Aussenseite der Gehäuseabdeckung (7) angeordnet und zusammen mit dem Metallfaservlies (8) von einem Ring (11) eingefasst ist.

5. Gasmelder nach einem der Ansprüche 1 bis 4, **dadurch gekennzeichnet, dass** in das Metallfaservlies (8) ein zusätzliches Stützgitter (9) integriert ist.

6. Gasmelder nach Anspruch 4, **dadurch gekennzeichnet, dass** das Stützgitter (10) und das Metallfaservlies (8) in den genannten Ring (11) eingegossen, eingeklebt oder eingespritzt sind.

7. Gasmelder nach Anspruch 6, **dadurch gekennzeichnet, dass** der Ring (11) mit der Gehäuseabdeckung (7) in das Gehäuse (4) eingepresst oder eingeklebt ist.

8. Gasmelder nach Anspruch 7, **dadurch gekennzeichnet, dass** der Ring (11) und der diesen tragende Gehäuseteil (4) aus PPO Noryl besteht.

9. Gasmelder nach einem der Ansprüche 1 bis 8, **dadurch gekennzeichnet, dass** das Metallfaservlies (8) eine Dicke von 1 mm und eine Porengrösse von 50 µm aufweist.

10. Gasmelder nach einem der Ansprüche 1 bis 9, **dadurch gekennzeichnet, dass** das Stützgitter (10) durch ein Quadratmaschengewebe mit einer Dicke von knapp 1 mm und einer Maschenweite von 0.5 bis 0.7 mm gebildet ist.

## Claims

1. Gas detector with pressure encapsulation for explosion protection, with a sensor housing (2) containing a sensor (5) and with a housing cover (7) embodied as a flame guard for shielding the sensor (5) against the environment and for making possible an exchange of gas with the latter, whereby the housing cover (7) is formed by a filter (8) made of non-woven metal material and a support grid (10).

2. Gas detector in accordance with claim 1, **characterized in that** the non-woven metal material (8) is made of chrome-nickel-steel.

3. Gas detector in accordance with claim 1 or 2, **characterized in that** the support grid (10) is made of chrome-nickel-steel.

4. Gas detector in accordance with claim 3, **characterized in that** the support grid (10) is arranged on the outside of the housing cover (7) and is enclosed together with the non-woven metal material (8) by a ring (11).

5. Gas detector in accordance with one of the claims 1 to 4, **characterized in that** an additional support grid (9) is integrated into the non-woven metal material (8).

6. Gas detector in accordance with claim 4, **characterized in that** the support grid (10) and the non-woven metal material (8) are encapsulated, glued or injection-moulded into said ring (11).

7. Gas detector in accordance with claim 6, **characterized in that** the ring (11) with the housing cover (7) is glued or pressed into the housing (4).

8. Gas detector in accordance with claim 7, **characterized in that** the ring (11) and housing part (4) supporting it are made of PPO Noryl.

9. Gas detector in accordance with one of the claims 1 to 8, **characterized in that** the non-woven metal material (8) has a thickness of 1 mm and a pore size of 50 µm.

10. Gas detector in accordance with one of the claims 1 to 9, **characterized in that** the support grid (10) is formed by square woven mesh with a thickness of around 1 mm and a mesh width of 0.5 to 0.7 mm.

## Revendications

1. Détecteur de gaz à enveloppe antidéflagrante, comprenant un boîtier de capteur (2) contenant un capteur (5) et comprenant un couvercle de boîtier (7) exécuté comme coupe-flamme pour la protection du capteur (5) contre l'environnement et pour la permission d'un échange de gaz avec celui-ci, le couvercle du boîtier (7) étant formé par un filtre (8) en non-tissé à fibres métalliques et par une grille de support (10).

2. Détecteur à gaz selon la revendication 1, **caractérisé en ce que** le non-tissé à fibres métalliques (8) est constitué d'acier au nickel-chrome.

3. Détecteur à gaz selon la revendication 1 ou 2, **caractérisé en ce que** la grille de support (10) est constituée d'acier au nickel-chrome.

4. Détecteur à gaz selon la revendication 3, **caractérisé en ce que** la grille de support (10) est disposée sur le côté extérieur du couvercle du boîtier (7) et est bordée, avec le non-tissé à fibres métalliques (8), par un anneau (11).

5. Détecteur à gaz selon l'une quelconque des revendications 1 à 4, **caractérisé en ce qu'**une grille de support supplémentaire (9) est intégrée dans le non-tissé à fibres métalliques (8).

6. Détecteur à gaz selon la revendication 4, **caractérisé en ce que** la grille de support (10) et le non-tissé à fibres métalliques (8) sont coulés, collés ou injectés dans ledit anneau (11).

7. Détecteur à gaz selon la revendication 6, **caractérisé en ce que** l'anneau (11) avec le couvercle du boîtier (7) est pressé ou collé dans le boîtier (4).

8. Détecteur à gaz selon la revendication 7, **caractérisé en ce que** l'anneau (11) et la partie du boîtier (4) portant celui-ci sont en PPO Noryl.

9. Détecteur à gaz selon l'une quelconque des revendications 1 à 8, **caractérisé en ce que** le non-tissé à fibres métalliques (8) présente une épaisseur de 1 mm et une taille de pores de 50 µm.

10. Détecteur à gaz selon l'une quelconque des revendications 1 à 9, **caractérisé en ce que** la grille de support (10) est formée par un tissu à mailles carrées ayant une épaisseur d'à peine 1 mm et une largeur de maille de 0,5 à 0,7 mm.
